(19) Europäisches Patentamt
European Patent Office
Office européen des brevets

(11) Numéro de publication : **0 375 510 B1**

(12) # FASCICULE DE BREVET EUROPEEN

(45) Date de publication du fascicule du brevet : **11.11.92 Bulletin 92/46**

(51) Int. Cl.$^5$ : **C07D 277/82,** A61K 31/425, C07D 417/12

(21) Numéro de dépôt : **89403459.4**

(22) Date de dépôt : **13.12.89**

(54) **Dérivés d'imino-2 polyfluoroalcoxy-6 benzothiazole, leurs procédés de préparation et les médicaments les contenant.**

Le dossier contient des informations techniques présentées postérieurement au dépôt de la demande et ne figurant pas dans le présent fascicule.

(30) Priorité : **15.12.88 FR 8816547**
**13.07.89 FR 8909482**

(43) Date de publication de la demande : **27.06.90 Bulletin 90/26**

(45) Mention de la délivrance du brevet : **11.11.92 Bulletin 92/46**

(84) Etats contractants désignés : **AT BE CH DE ES FR GB GR IT LI LU NL SE**

(56) Documents cités :
**EP-A- 282 971**
**EP-A- 0 050 551**
**FR-A- 2 357 553**
**Neuroscience Letters 1988 (88) (2) 195 - 200**

(73) Titulaire : **RHONE-POULENC SANTE**
**20, avenue Raymond Aron**
**F-92160 Antony (FR)**

(72) Inventeur : **Audiau, François**
**9 rue Guérin**
**F-94220 Charenton le Pont (FR)**
Inventeur : **Gueremy, Claude**
**3 rue Daumesnil**
**F-78800 Houilles (FR)**
Inventeur : **Jimonet, Patrick**
**13 Avenue de Normandie**
**F-78450 Villepreux (FR)**
Inventeur : **Mignani, Serge**
**14 Allée Jean-Baptiste Clément**
**F-93190 Livry-Gargan (FR)**

(74) Mandataire : **Savina, Jacques et al**
**RHONE-POULENC RORER S.A., Direction des Brevets, 90 avenue Raymond Aron**
**F-92165 Antony Cédex (FR)**

EP 0 375 510 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des composés de formule :

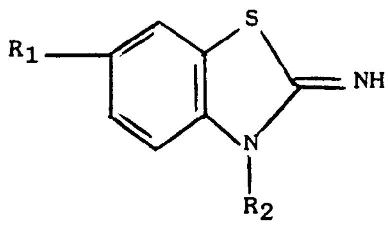

(I)

leurs sels, leurs procédés de préparation et les médicaments les contenant.

Le brevet EP 50551 décrit l'amino-2 trifluoromethoxy-6 benzothiaziole comme anxiolytique, anticonvulsivant et hypnotique.

Ce produit et certains dérivés d'amino-2 benzothiazole sont également connus comme antagonistes du glutamate (Neuroscience letters, 88,195 (1988) et demande de brevet EP 28971).

Dans la formule (I),

- $R_1$ représente un radical polyfluoroalcoxy et
- $R_2$ représente un radical alkyle, alcényle (3-6 C), cycloalkyl (3-6 C) alkyle, carbamoylalkyle, dialkylcarbamoylalkyle, acylaminoalkyle, phénylthioalkyle, hydroxyalkyle, cyanoalkyle, sulfamoyléthyle, N-alkylsulfamoyléthyle, pyridylthioalkyle, pyridylsulfinylalkyle, alcynyle (3-6 C), phénylsulfinylalkyle, halogénophénylthioalkyle, (trifluoro-2,2,2 éthyl) thioalkyle, pyrimidinylsulfinylalkyle, pyridylalkylsulfinylalkyle, halogénophénylsulfinylalkyle ou (trifluoro2,2,2 éthyl) sulfinylalkyle.

Sauf mention contraire, dans les définitions qui précédent et celles qui sont citées ci-après, les radicaux alkyle et les portions alkyle et alcoxy contiennent 1 à 4 atomes de carbone en chaîne droite ou ramifiée.

L'invention concerne également les sels d'addition des composés de formule (I) avec les acides minéraux ou organiques.

Les radicaux polyfluoroalcoxy préférés sont les radicaux trifluorométhoxy, pentafluoroéthoxy, trifluoro-2,2,2 éthoxy ou tétrafluoro-1,1,2,2 éthoxy ou pentafluoro-2,2,3,3,3 propoxy.

Les composés de formule (I) pour lesquels $R_2$ représente un radical cyanoalkyle, carbamoylalkyle, sulfamoyléthyle ou N-alkylsulfamoyléthyle peuvent être obtenus par action de brome et d'un thiocyanate de métal alcalin sur un dérivé de formule :

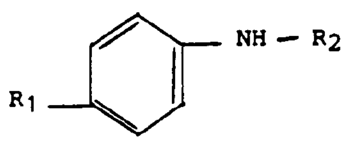

(II)

dans laquelle $R_1$ a les mêmes significations que dans la formule (I) et $R_2$ a les mêmes significations que précédemment.

Cette réaction s'effectue généralement au sein de l'acide acétique, à une température d'environ 20°C.

Comme thiocyanate de métal alcalin, on utilise de préférence le thiocyanate de potassium.

Les dérivés de formule (II) pour lesquels $R_2$ représente un radical cyanoalkyle peuvent être obtenus par action d'une polyfluoroalcoxy-4 aniline avec un dérivé de formule :

$$Hal — R_2 \qquad (III)$$

dans laquelle hal représente un atome d'halogène et $R_2$ représente un radical cyanoalkyle.

Cette réaction s'effectue généralement au sein d'un solvant organique inerte ou de l'eau, à une température comprise entre 50°C et la température d'ébullition du solvant.

Les polyfluoroalcoxy-4 anilines peuvent être obtenues par application ou adaptation des méthodes décrites par W.A. SHEPPARD, J. Org. Chem., 29,1(1964) ; dans le Beilstein 12,1166 et dans les brevets US 3 920 444, US 2436100, DE 3195926, DE 2606982 et EP 205821.

Les dérivés de formule (II) pour lesquels $R_2$ représente un radical carbamoylalkyle peuvent être obtenus à partir des nitriles correspondants par toute méthode connue de l'homme de l'art permettant de passer d'un nitrile à un amide.

On opère de préférence au moyen d'acide sulfurique, à une température comprise entre 60°C et 100°C.

Les dérivés de formule (II) pour lesquels $R_2$ représente un radical sulfamoyléthyle ou N-alkylsulfamoyléthyle peuvent être obtenus par action d'un dérivé de formule :

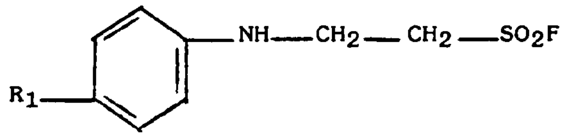

(IV)

dans laquelle $R_1$ a les mêmes significations que dans la formule (I) sur l'hydroxyde d'ammonium ou une alkylamine.

Cette réaction s'effectue généralement au sein d'un solvant inerte tel qu'une cétone (acétone, méthyléthylcétone...) ou un solvant aromatique (benzène, toluène...), à une température comprise entre 30°C et la température d'ébullition du solvant.

Le dérivés de formule (IV) peuvent être obtenus par action d'une polyfluoroalcoxy-4 aniline sur le fluorure de vinylsulfonyle.

Cette réaction s'effectue de préférence au sein d'un solvant inerte tel que le diméthylformamide, à une température voisine de 20°C.

Le fluorure de vinylsulfonyle peut être obtenu selon la méthode décrite par J.J. KRUTAK et coll., J. Org. Chem., 44(22), 3847(1979).

Les dérivés de formule (I) pour lesquels $R_2$ représente un radical pyridylthioalkyle ou pyridylalkylthioalkyle peuvent être préparés par action d'un dérivé de formule :

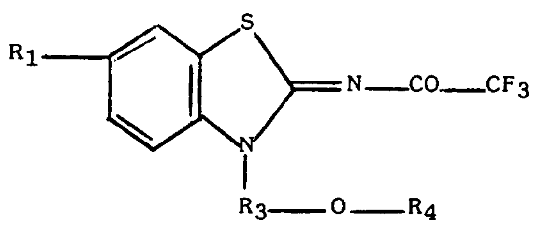

(V)

dans laquelle $R_1$ a les mêmes significations que dans la formule (I), $R_3$ représente un radical alkylène (1-4 C) et $R_4$ représente un groupe réactif tel qu'un radical méthanesulfonyle ou p-toluènesulfonyle sur une mercaptopyridine puis hydrolyse du produit obtenu.

Cette réaction s'effectue généralement au sein d'un solvant inerte tel que le diméthylformamide, à une température voisine de 20°C. L'hydrolyse s'effectue au moyen d'une base telle que l'ammoniaque concentrée, à la température d'ébullition du milieu réactionnel.

Les dérivés de formule (V) peuvent être préparés par action d'un dérivé de formule :

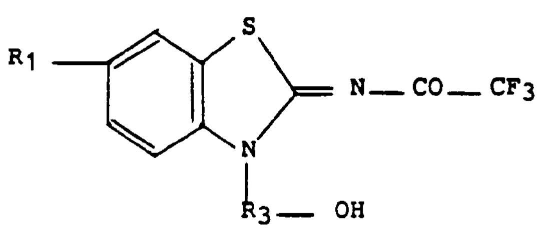

(VI)

dans laquelle $R_1$ et $R_3$ ont les mêmes significations que dans la formule (V) sur le chlorure d'acide méthanesulfonique ou le chlorure d'acide p-toluènesulfonique.

Cette réaction s'effectue de préférence soit au sein d'un solvant inerte tel que le benzène, le toluène, le chloroforme, le chlorure de méthylène, en présence d'une amine tertiaire telle que la triéthylamine, à une température voisine de 20°C, soit au sein de la pyridine à une température voisine de 0°C.

Les dérivés de formule (VI) peuvent être obtenus par action d'un dérivé de formule :

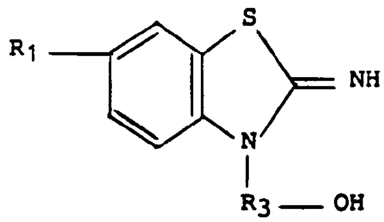

(VII)

dans laquelle $R_1$ et $R_3$ ont les mêmes significations que dans la formule (V) sur le trifluoroacétate d'éthyle.

Cette réaction s'effectue généralement au sein d'un alcool, en présence d'une base tertiaire telle que la triéthylamine, à une température voisine de 20°C.

Les dérivés de formule (VII) peuvent être obtenus par action d'un amino-2 polyfluoroalcoxy-6 benzothiazole sur un alcool de formule :

$$Hal—R_3—OH \qquad (VIII)$$

dans laquelle Hal représente un atome d'halogène et $R_3$ a les mêmes significations que dans le formule (VII).

Cette réaction s'effectue au sein d'un alcool, à la température d'ébullition du solvant.

Les amino-2 polypolyfluoroalcoxy-6 benzothiazole peuvent être préparés par application ou adaptation de la méthode décrite par L.M YAGUPOL'SKII et coll., Zh. Obsch. Khim., 33(7), 2301(1963).

Les composés de formule (I) pour lesquels $R_2$ représente un radical alkyle, alcènyle, cycloalkylalkyle, dialkylcarbamoylalkyle, acylaminoalkyle, phénylthioalkyle, hydroxyalkyle, alcynyle, halogénophénylthioalkyle ou, (trifluoro-2,2,2 éthyl) thioalkyle peuvent être préparés par action d'un dérivé aminé de formule :

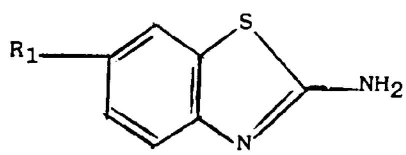

(IX)

sur un dérivé de formule :

$$R_2 - X \qquad (X)$$

dans lesquelles $R_2$ a les mêmes significations que précédemment, $R_1$ a les mêmes significations que dans la formule (I) et X représente un groupe réactif tel qu'un radical tosyloxy ou un atome d'halogène (chlore, brome, iode de préférence).

Cette réaction s'effectue de préférence au sein d'un solvant inerte tel qu'un alcool (éthanol, propanol par exemple), une cétone (acétone, méthyléthylcétone par exemple) ou le diméthylformamide, à une température comprise entre 10°C et la température d'ébullition du solvant, éventuellement en présence d'iodure de sodium et éventuellement après fusion à 130-140°C des composés de formules (IX) et (X).

Les dérivés de formule (X) pour lesquels $R_2$ représente un radical alcynyle peuvent être obtenus par application ou adaptation des méthodes décrites dans le Beilstein, 1,IV, 970 et 974.

Les dérivés de formule (X) pour lesquels $R_2$ représente un radical halogènophénylthioalkyle peuvent être obtenus par application ou adaptation de la méthode décrite par H.P.S. CHAWLA et coll., J. Med. Chem., 13(3), 480(1970).

Les dérivés de formule (X) pour lesquels $R_2$ représente un radical (trifluoro-2,2,2 éthyl) thioalkyle peuvent être obtenus par action d'un dérivé de formule :

$$HO—R_2 \qquad (XI)$$

dans laquelle $R_2$ a les mêmes significations que précédemment sur le tétrachlorure de carbone, en présence de triphénylphosphine.

Cette réaction s'effectue généralement à la température d'ébullition du milieu réactionnel.

Les dérivés de formule (XI) pour lesquels $R_2$ représente un radical (trifluoro-2,2,2 éthyl) thioalkyle peuvent être obtenus par application ou adaptation de la méthode décrite par R.C. TERRELL et coll., J. Org. Chem., 30(12), 4011(1965).

Les autres dérivés de formule (X) qui ne sont pas commercialisés peuvent être préparés par application ou adaptation de la méthode décrite par W.C. HOWELL, J. Amer. Chem. Soc., 78, 3843 (1956) et des méthodes décrites dans les exemples.

Les composés de formule (I) pour lesquels $R_2$ représente un radical pyridylalkylsulfinylalkyle, phénylsulfinylalkyle, pyridylsulfinylalkyle, pyrimidinylsulfinylalkyle, halogénophénylsulfinylalkyle ou (trifluoro-2,2,2 éthyl) sulfinylalkyle peuvent être préparés par oxydation des dérivés correspondants pour lesquels $R_2$ représente un radical pyridylalkylthioalkyle, phénylthioalkyle, pyridylthioalkyle, pyrimidinylthioalkyle, halogénophénylthioalkyle ou (trifluoro-2,2,2 éthyl) thioalkyle.

Cette oxydation peut s'effectuer au moyen d'acide m-chloroperbenzoïque, au sein d'un alcool, à une température voisine de 20°C.

Les dérivés pour lesquels $R_2$ représente un radical phénylthioalkyle peuvent être préparés par action d'une amino-2 polyfluoroalcoxy-6 benzothiazole sur un halogénoalkylthioalkyle.

Cette réaction s'effectue généralement au sein d'un solvant organique tel que l'éthanol, le propanol, la méthyléthylcétone ou le diméthylformamide, à une température comprise entre 60°C et la température d'ébullition du solvant.

Les dérivés pour lesquels $R_2$ représente un radical pyrimidinylthioalkyle peuvent être préparés par action d'une mercaptopyrimidine sur un dérivé de formule (V).

Les mélanges réactionnels obtenus par les divers procédés décrits précédemment sont traités suivant des méthodes classiques physiques (évaporation, extraction, distillation, cristallisation, chromatographie...) ou chimiques (formation de sels...).

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés sont actifs vis-à-vis des convulsions induites par le glutamate et sont donc utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyotrophique latérale et l'atrophie olivopontocérébelleuse.

L'activité des composés de formule (I) vis-à-vis des convulsions induites par le glutamate a été déterminée selon une technique inspirée de celle de I.P. LAPIN, J. Neural. Transmission, vol.54, 229-238 (1982) ; l'injection du glutamate par voie intracérébroventriculaire étant effectuée selon une technique inspirée de celle de R. CHERMAT et P. SIMON, J. Pharmacol. (Paris), vol.6, 489-492 (1975). Leur $DE_{50}$ est égale ou inférieure à 10 mg/kg.

Les composés de formule (I) présentent une toxicité faible. Leur $DL_{50}$ est supérieure à 15 mg/kg par voie I.P. chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, isothionate, théophylline-acétate, salicylate, sulfate, nitrate et phosphate.

Les exemples suivants, donnés à titre non limitatif montrent comment l'invention peut-être mise en pratique.

## EXEMPLE 1

A 3g de (trifluorométhoxy-4 anilino)-3 propionitrile et 7,8g de thiocyanate de potassium dissous dans 50 cm3 d'acide acétique est additionné à température ambiante 3,2g (1 cm3) de brome. L'agitation est maintenue, pendant 12 heures, à cette température. Le mélange est évaporé à sec à 80°C sous pression réduite (20 mm de mercure ; 2,7 KPa). Le résidu obtenu est repris par 100 cm3 d'eau et le pH est amené à 9-10 par addition de soude concentrée (10N). Après extraction par deux fois 50 cm3 d'acétate d'éthyle, lavage des phases réunies par deux fois 20 cm3 d'eau, séchage sur du sulfate de magnésium anhydre et évaporation à sec à 40°C sous pression réduite (20 mm de mercure ; 2,7 KPa) on isole une huile brune. On dissout cette huile dans 100 cm3 d'acétone et on ajoute 2g d'acide oxalique. On isole ainsi 5,5g d'imino-2(cyano-2 éthyl)-3 trifluorométhoxy-6 benzothiazoline sous forme de monooxalate fondant à 180°C.

Le (trifluorométhoxy-4 anilino)-3 propionitrile peut être préparé de la façon suivante : 17,7g de trifluorométhoxy-4 aniline, 6,7g de bromo-3 propionitrile dans 20 cm3 d'eau sont portés à reflux sous agitation pendant 12 heures. La solution est ensuite refroidie et amenée à sec à 80°C sous pression réduite (20 mm de mercure ; 2,7 KPa). Le résidu obtenu est purifié par flash-chromathographie sur colonne de silice sous courant d'azote, à moyenne pression (0,5-1,5 bar) avec un mélange de cyclohexane et d'acétate d'éthyle (80-20 en volumes) comme éluant. On obtient 8,7g d'une huile jaune.

EXEMPLE 2

On opère comme dans l'exemple 1, à partir de 4,2g de (trifluorométhoxy-4 anilino)-3 propionamide,de 6,6g de thiocyanate de potassium et de 2,7g (0,85 cm3) de brome dans 50 cm3 d'acide acétique. Le mélange est agité, à une température d'environ 20°C, pendant 12 heures. On ajoute 100 cm3 d'eau et on amène le pH à 9-10 par de la soude concentrée (10N). Après extraction par deux fois 50 cm3 d'acétate d'éthyle, séchage des phases organiques réunies sur du sulfate de magnésium anhydre et évaporation à sec à 40°C sous pression réduite (20 mm de mercure ; 2,7 kPa) on isole un solide qui est recristallisé dans 100 cm3 d'acétonitrile bouillant. On obtient 2,3g d'(imino-2 trifluorométhoxy-6 benzothiazolinyl-3)-3 propionamide fondant à 219°C.

Le (trifluorométhoxy-4 anilino)-3 propionamide peut être préparé de la façon suivante : 5,4g de (trifluorométhoxy-4 anilino)-3 propionitrile obtenu dans l'exemple 1 et 20 cm3 d'acide sulfurique concentré sont chauffés à 90°C pendant 2 heures. Après refroidissement à une température de 20°C, on ajoute cette solution dans 250g de glace et on amène le pH à 9-10 par de la soude concentrée (10N). On isole ainsi directement 4,2g de (trifluorométhoxy-4 anilino)-3 propionamide fondant à 76°C.

EXEMPLE 3

A un mélange de 4,1g de (p-trifluorométhoxy anilino)-2 éthanesulfonamide et de 5,6g de thiocyanate de potassium dans 20 cm3 d'acide acétique, on ajoute, en environ 15 minutes, 2,3g de brome en solution dans 10 cm3 du même solvant. La réaction est poursuivie 15 heures à une température voisine de 20°C. Après addition de 50 cm3 d'eau distillée, le milieu réactionnel est neutralisé par de la soude à 30%. Le précipité formé est filtré puis repris dans 50 cm3 de méthanol, à nouveau filtré et le filtrat concentré à sec sous pression réduite (20 mm de mercure; 2,7kPa). Après formation du chlorhydrate par addition de 3,6 cm3 d'éther chlorhydrique 4,2N dans un mélange d'éther éthylique et de méthanol, puis recristallisation dans 50 cm3 d'éthanol absolu, on obtient 2,9g de chlorhydrate d'(imino-2 trifluorométhoxy-6 benzothiazolinyl-3)-2 éthanesulfonamide sublimant vers 225°C.

Le (p-trifluorométhoxy anilino)-2 éthanesulfonamide peut être préparé selon le procédé suivant: 8,6g de fluorure de (p-trifluorométhoxy anilino)-2 éthanesulfonyle et 30 cm3 d'hydroxyde d'ammonium à 28% dans 50 cm3 d'acétone sont chauffés à ébullition pendant 1 heure. Après refroidissement à une température voisine de 20°C, l'acétone est évaporée sous pression réduite (20 mm de mercure; 2,7kPa) et la phase organique extraite par 3 fois 50 cm3 d'acétate d'éthyle. Après séchage sur sulfate de magnésium, concentration à sec sous pression réduite puis chromatographie sur colonne de silice avec un mélange d'acétate d'éthyle et de cyclohexane (50-50 en volumes) comme éluant, on obtient 5,5g de (p-trifluorométhoxy anilino)-2 éthanesulfonamide sous forme d'une huile rosée.

Le fluorure de (p-trifluorométhoxy anilino)-2 éthanesulfonyle peut être préparé de la manière suivante: A 19,1g de p-trifluorométhoxy aniline en solution dans 20 cm3 de diméthylformamide, on ajoute goutte à goutte 11,9g de fluorure de vinylsulfonyle en solution dans 10 cm3 de diméthylformamide. La réaction est poursuivie 2 heures à une température voisine de 20°C. Le milieu réactionnel est ajouté à 300 cm3 d'eau distillée et la phase organique extraite par 3 fois 50 cm3 d'éther éthylique. Après séchage et concentration à sec sous pression réduite (20 mm de mercure; 2,7 kPa), on obtient 25,8g de fluorure de (p-trifluorométhoxy anilino)-2 éthanesulfonyle sous forme d'une huile orangée utilisée à l'état brut dans les étapes de synthèse ultérieures.

Le fluorure de vinylsulfonyle peut être obtenu selon la méthode décrite par J.J.Krutak et coll., J.Org.Chem. 44 (22) 3847 (1979).

EXEMPLE 4

On opère comme à l'exemple 3, à partir de 3,5g de N-méthyl (p-trifluorométhoxy anilino)-2 éthanesulfonamide, de 4,7g de thiocyanate de potassium et de 2,2g de brome dans 30 cm3 d'acide acétique. Après 18 heures à une température voisine de 20°C, neutralisation par de la soude à 30% et extraction par de l'acétate d'éthyle on obtient un produit brut qui est transformé en chlorhydrate par addition de 3 cm3 d'éther chlorhydrique 4,2N dans 25 cm3 d'éthanol et recristallisé dans 50 cm3 d'éthanol absolu. Le chlorhydrate de N-méthyl (imino-2 trifluorométhoxy-6 benzothiazolinyl-3)-2 éthanesulfonamide ainsi obtenu (2,1g) fond à 242°C.

Le N-méthyl (p-trifluorométhoxy anilino)-2 éthanesulfonamide peut être préparé de la manière suivante : On opère comme à l'exemple 3 pour la préparation du (p-triluorométhoxy anilino)-2 éthanesulfonamide mais à partir de 6,0g de fluorure de (p-trifluorométhoxy anilino)-2 éthanesulfonyle et de 20 cm3 de méthylamine aqueuse à 40% dans 30 cm3 d'acétone. Après chauffage 1 heure à ébullition, l'acétone est évaporée et la phase organique extraite par de l'acétate d'éthyle. Le produit brut obtenu est purifié par chromatographie sur colonne de silice avec un mélange d'acétate d'éthyle et de cyclohexane (50-50 en volumes) comme éluant. On

obtient 3,5g de N-méthyl (p-trifluorométhoxy anilino)-2 éthanesulfonamide sous forme d'une huile jaune utilisée à l'état brut dans les réactions suivantes.

EXEMPLE 5

On opère comme à l'exemple 3, à partir de 6,1g de N-éthyl (p-trifluorométhoxy anilino)-2 éthanesulfonamide, de 7,6g de thiocyanate de potassium et de 3,6g de brome dans 60 cm3 d'acide acétique. Après 18 heures à une température voisine de 20°C, neutralisation par de la soude à 30% et extraction par de l'acétate d'éthyle on obtient un produit brut qui est transformé en chlorhydrate par addition de 5 cm3 d'éther chlorhydrique 4,2N dans 30 cm3 d'éthanol et recristallisé dans 50 cm3 d'éthanol absolu. On obtient 3,0g de chlorhydrate de N-éthyl (imino-2 trifluorométhoxy-6 benzothiazolinyl-3 )-2 éthanesulfonamide ainsi obtenu (3,0g) fond à 240°C.

Le N-éthyl (p-trifluorométhoxy anilino)-2 éthanesulfonamide peut être préparé comme dans l'exemple 3 pour la préparation du (p-trifluorométhoxy anilino)-2 éthanesulfonamide, à partir de 6,0g de fluorure de (p-trifluorométhoxy anilino)-2 éthanesulfonyle et de 20 cm3 d'éthylamine aqueuse à 33% dans 30 cm3 d'acétone. Après chauffage 1 heure à ébullition, l'acétone est évaporée et la phase organique extraite par de l'acétate d'éthyle. On obtient 6,1g de N-éthyl (p-trifluorométhoxy anilino)-2 éthanesulfonamide sous forme d'une huile brune utilisée à l'état brut dans les réactions suivantes.

EXEMPLE 6

A une solution de sel de sodium de la mercapto-4 pyridine (obtenue après cessation du dégagement gazeux, d'une solution de 1,8g de mercapto-4 pyridine dans 30 cm3 de diméthylformamide et d'une suspension de 0,8g d'hydrure de sodium en dispersion à 50% dans l'huile de vaseline, pendant 1 heure à 50°C) refroidie à une température proche de 20°C, on ajoute 8g de para-toluènesulfonate de (trifluoroacétylimino-2 trifluorométhoxy-6 benzothiazolinyl-3)-2 éthyle. Le milieu réactionnel est agité pendant deux heures à une température voisine de 20°C. On ajoute ensuite à cette solution dans l'ordre 50 cm3 d'éthanol, 10 cm3 d'eau et 20 cm3 d'ammoniaque concentrée (10N). La solution est portée au reflux pendant 1 heure et refroidie à une température voisine de 20°C. Cette solution aqueuse est extraite par deux fois 50 cm3 de dichlorométhane ; la phase organique est séchée sur sulfate de magnésium anhydre, filtrée et on concentrée à sec à 40°C sous pression réduite (20 mm de mercure ; 2,7 PKa). Le résidu obtenu est purifié par flash-chromatographie sur colonne de silice, sous courant d'azote, à moyenne pression (0,5-1,5 bar) avec de l'acétate d'éthyle comme éluant et le solide obtenu est traité avec 2,4g d'acide oxalique et 10 cm3 d'acétone. On isole 3,5g d'imino-2 [(pyridyl-4 thio)-2 éthyl]-3 trifluorométhoxy-6 benzothiazoline sous forme de dioxalate fondant à 164°C.

Le p-toluènesulfonate de (trifluoroacétylimino-2 trifluorométhoxy-6 benzothiazolinyl-3)-2 éthyle peut être préparé selon le procédé suivant : 19,3g de (trifluoroacétylimino-2 trifluorométhoxy-6 benzothiazolinyl-3)-2 éthanol sont ajoutés progressivement à 19,7g de chlorure de p-toluènesulfonyle en solution dans 120 cm3 de pyridine refroidie à 0°C. La réaction est poursuivie 1 heure à 10-15°C. Le milieu réactionnel est ajouté à 500 cm3 d'eau distillée et la phase organique extraite par 3 fois 100 cm3 de dichlorométhane. Après lavage par 2 fois 50 cm3 d'acide chlorhydrique 1N, puis 2 fois 50 cm3 d'eau distillée, séchage sur sulfate de magnésium et concentration à sec sous pression réduite (20 mm de mercure ; 2,7kPa), on obtient 14,1g de p-toluènesulfonate de (trifluoroacétylimino-2 trifluorométhoxy-6 benzothiazolinyl-3)-2 éthanol fondant à 143°C.

Le (trifluoroacétylimino-2 trifluorométhoxy-6 benzothiazolinyl-3)-2 éthanol, peut être préparé de la manière suivante : 20,7g de bromhydrate d'(imino-2 trifluorométhoxy-6 benzothiazolinyl-3)-2 éthanol, 9,8g de trifluoroacétate d'éthyle et 16,1 cm3 de triéthylamine sont agités dans 100 cm3 d'éthanol pendant 22 heures à une température voisine de 20°C. Après concentration à sec sous pression réduite, le résidu obtenu est purifié par chromatographie sur colonne de silice avec de l'acétate d'éthyle comme éluant. On obtient 19,2g de (trifluoroacétylimino-2 trifluorométhoxy-6 benzothiazolinyl-3)-2 éthanol fondant à 144°C.

L'(imino-2 trifluorométhoxy-6 benzothiazolinyl-3)-2 éthanol peut être préparé selon le procédé suivant : 9,4g d'amino-2 trifluorométhoxy-6 benzothiazole et 10g de bromo-2 éthanol dans 30 cm3 d'éthanol absolu sont chauffés pendant 95 heures à ébullition. Le mélange est ensuite refroidi à une température voisine de 20°C. Le précipité formé est filtré et lavé avec 100 cm3 d'éther éthylique. On obtient 6,4g de bromhydrate d'(imino-2 trifluorométhoxy-6 benzothiazolinyl-3)-2 éthanol fondant à 219°C.

L'amino-2 trifluorométhoxy-6 benzothiazole peut-être préparé selon la méthode décrite par L.M. YAGUPOL'SKII et Coll., Zh. Obahch. Khim, 33(7), 2301(1963).

EXEMPLE 7

A 1,9g d'imino-2 [(pyridyl-2 thio)-2 éthyl]-3 trifluorométhoxy-6 benzothiazoline dissous dans 30 cm3 de

chloroforme on ajoute à une température voisine de -10°C, sous agitation, 1g d'acide méta-chloroperbenzoique (à 90% en poids) en dix minutes. Le mélange réactionnel est agité 1 heure à une température voisine de 20°C puis concentré à 40°C sous pression réduite (20 mm de mercure, 2,7 KPa). Le résidu est purifié par flash-chromatographie sur colonne de silice, sous courant d'azote, à moyenne pression (0,5-1,5 bar) avec un mélange d'acétate d'éthyle et de cyclohexane (70-30 en volumes) comme éluant. L'huile ainsi obtenue est reprise par 50 cm3 d'éther diéthylique et on isole 1g d'imino-2 [(pyridyl-2 sulfinyl)-2 éthyl]-3 trifluorométhoxy-6 benzothiazoline-(RS) fondant à 94°C.

L'imino-2 [(pyridyl-2 thio)-2 éthyl]-3 trifluorométhoxy-6 benzothiazoline peut être préparée de la façon suivante : on opère comme dans l'exemple 6, à partir de 1,6g d'hydrure de sodium à 50% en dispersion dans l'huile de vaseline, de 3,6g de mercapto-2 pyridine, de 16g de para-toluènesulfonate de (trifluoroacétylimino-2 trifluorométhoxy-6 benzothiazolinyl-3)-2 éthyle et de 30 cm3 de diméthylformamide. Le mélange est agité 12 heures à une température proche de 20°C. On ajoute ensuite à cette solution dans l'ordre 50 cm3 d'éthanol, 10 cm3 d'eau et 20 cm3 d'ammoniaque concentrée (10N). La solution est portée au reflux pendant 1 heure et refroidie à une température voisine de 20°C. On extrait cette solution aqueuse par deux fois 50 cm3 de dichlorométhane, on sèche sur sulfate de magnésium anhydre, on filtre et on concentre à sec à 40°C sous pression réduite (20 mm de mercure ; 2,7 PKa). Le résidu est repris par 40 cm3 d'éther isopropylique et 80 cm3 d'hexane. On isole ainsi directement 8g d'imino-2 [(pyridyl-2 thio)-2 éthyl]-3 trifluorométhoxy-6 benzothiazoline fondant à 104°C.

EXEMPLE 8

A 1,3g d'imino-2 (phénylthio-2 éthyl)-3 trifluorométhoxy-6 benzothiazoline dans 20 cm3 d'éthanol absolu refroidi à 0°C, on ajoute 0,6g d'acide m-chloroperbenzoïque en environ 10 minutes. La réaction est poursuivie 3 heures à une température voisine de 20°C. Le milieu réactionnel est concentré à sec sous pression réduite (20 mm de mercure; 2,7kPa) et le résidu obtenu purifié par chromatographie sur colonne de silice avec de l'acétate d'éthyle comme éluant. On obtient 0,7g d'imino-2 (phénylsulfinyl-2 éthyl)-3 trifluorométhoxy-6 benzothiazoline-(RS) sous forme d'une huile jaunâtre que l'on transforme en chlorhydrate fondant à 210°C.

EXEMPLE 9

On chauffe à reflux pendant 6 heures 10 g de bromo-1 butyne-2, 17 g d'amino-2 trifluorométhoxy-6 benzothiazole et 30 cm3 d'éthanol. Après retour à une température voisine de 20°C, l'éthanol est évaporé sous pression réduite (20 mm de mercure; 2,7 kPa) et le solide rouge obtenu est repris avec 100 cm3 d'eau, alcalinisé avec de l'ammoniaque à 28% et extrait avec 300 cm3 d'acétate d'éthyle au total. L'extrait organique est séché sur sulfate de magnésium, filtré et évaporé sous pression réduite (20 mm de mercure; 2,7 kPa). Le résidu d'évaporation est purifié par chromatographie sur colonne de silice avec comme éluant un mélange de cyclohexane et d'acétate d'éthyle (60-40 en volumes): on obtient un solide crème (7,2 g) qui est recristallisé dans un mélange de 100 cm3 de cyclohexane et 5 cm3 d'acétate d'éthyle pour fournir 5,1 g de (butyne-2 yl)-3 imino-2 trifluorométhoxy-6 benzothiazoline fondant à 116°C.

Le bromo-1 butyne-2 peut-être préparé selon la méthode décrite dans le BEILSTEIN 1, IV 974.

L'amino-2 trifluorométhoxy-6 benzothiazole peut-être obtenu selon la méthode décrite par L.M. YAGUPOL'SKII et coll., Zh. Obsch. Khim, 33(7), 2301(1963).

EXEMPLE 10

On opère comme à l'exemple 9, à partir de 16 g de bromo-4 butyne-1 et 16 g d'amino-2 trifluorométhoxy-6 benzothiazole que l'on chauffe à reflux pendant 5 heures. Après purification par chromatographie sur colonne de silice avec comme éluant un mélange de cyclohexane et d'acétate d'éthyle (50-50 en volumes), on obtient un solide blanc (1g) qui est trituré dans 6 cm3 d'éther de pétrole (40-65°C), filtré et séché à 40°C sous pression réduite (3 mm de mercure; 0,4 kPa) pour donner finalement 0,7 g de (butyne-3 yl)-3 imino-2 trifluorométhoxy-6 benzothiazoline fondant à 73°C.

Le bromo-4 butyne-1 peut être préparé selon la méthode décrite dans le BEILSTEIN 1, IV 970.

EXEMPLE 11

Un mélange de 9,4g d'amino-2 trifluorométhoxy-6 benzothiazole, de 15,2g de chloro-1 (fluoro-4 phénylthio)-2 éthane et de 12g d'iodure de sodium dans 30 cm3 de méthyléthylcétone est chauffé 48 heures à ébullition. Après refroidissement à une température voisine de 20°C, on ajoute 50 cm3 d'éther éthylique et on filtre

le précipité obtenu. Ce dernier est repris dans 100 cm3 d'eau distillée et traité par 10 cm3 de soude 1N. Après extraction par 100 cm3 de dichlorométhane, séchage sur sulfate de magnésium et concentration sous pression réduite (20 mm de mercure; 2,7 kPa), on obtient 1,4g d'imino-2 [(fluoro-4 phénylthio-2) éthyl]-3 imino-2 trifluorométhoxy-6 benzothiazoline sous forme d'une huile jaune que l'on transforme en chlorhydrate fondant à 200°C.

Le chloro-1 (fluoro-4 phénylthio)-2 éthane peut être préparé selon la méthode décrite par H.P.S. CHAWLA et coll., J.Med. Chem., 13 (3), 480 (1970).

EXEMPLE 12

Un mélange de 5,9g d'amino-2 trifluorométhoxy-6 benzothiazole, de 4,8g de chloro-1 (trifluoro-2,2,2 éthyl)thio-2 éthane et de 3,8g d'iodure de sodium dans 20 cm3 de méthyléthylcétone est chauffé 72 heures à ébullition puis refroidi à une température voisine de 20°C. Après addition de 100 cm3 d'éther éthylique, le précipité formé est filtré et le filtrat concentré à sec sous pression réduite (20 mm de mercure; 2,7kPa). Le résidu obtenu est repris dans 20 cm3 d'acétate d'éthyle et le chlorhydrate formé par addition de 7 cm3 d'éther chlorhydrique 4,2N. On obtient 1,2g de chlorhydrate d'imino-2 (trifluoro-2,2,2 éthyl)thio-2 éthyl)-3 trifluorométhoxy-6 benzothiazoline sublimant vers 180°C.

Le chloro-1 (trifluoro-2,2,2 éthyl)thio-2 éthane peut être préparé selon le procédé suivant: 13,1g de (trifluoro-2,2,2 éthyl) thio-2 éthanol et 27,8g de triphénylphosphine dans 60 cm3 de tétrachlorure de carbone sont chauffés 3 heures à ébullition. Après refroidissement à 0°C, 70 cm3 de cyclohexane sont ajoutés, le précipité formé est filtré et le filtrat concentré à sec sous pression réduite (20 mm de mercure; 2,7kPa). Le résidu obtenu est purifié par distillation. On obtient 4,8g de chloro-1 (trifluoro-2,2,2 éthyl)thio-2 éthane dont le point d'ébullition est de 105°C sous 200 mm de mercure.

Le (trifluoro-2,2,2 éthyl)thio-2 éthanol peut être préparé selon la méthode suivante: 6,1g de sodium sont ajoutés progressivement à 170 cm3 d'éthanol absolu à une température voisine de 20°C. A l'éthylate de sodium ainsi formé on additionne 18,6 cm3 de mercapto-2 éthanol en environ 30 minutes, puis 25,9 cm3 d'iodure de trifluoroéthyle. Le mélange réactionnel est porté à ébullition pendant 30 minutes puis concentré à sec sous pression réduite. Le résidu obtenu est repris par 300 cm3 d'éther éthylique, le précipité formé est filtré et le filtrat lavé par 3 fois 200 cm3 d'eau distillée. Après séchage sur sulfate de magnésium et concentration à sec sous pression réduite, on obtient 22,6g de (trifluoro-2,2,2 éthyl)thio-2 éthanol sous forme d'une huile incolore.

EXEMPLE 13

7,1 g d'amino-2 trifluorométhoxy-6 benzothiazole et 4,3 g d'iodure de méthyle dans 20 cm3 d'éthanol absolu sont chauffés pendant 18 heures à ébullition. Le mélange est ensuite refroidi à une température voisine de 20°C. Le précipité formé est séparé par filtration et lavé par 2 fois 20 cm3 d'éthanol absolu. Le solide est repris dans 100 cm3 d'eau distillée chauffée à 60°C et la solution obtenue est traitée par 2,6 g d'hydrogénocarbonate de sodium. Le précipité est séparé par filtration et recristallisé dans 50 cm3 d'un mélange d'éthanol absolu et d'eau distillée (50-50 en volumes) bouillant. On obtient 2,1 g d'imino-2 méthyl-3 trifluorométhoxy-6 benzothiazoline fondant à 60-62°C.

L'amino-2 trifluorométhoxy-6 benzothiazole peut être préparé selon la méthode décrite par L.M. YAGUPOL'SKII et coll., Zh. Obshch. Khim., 33(7), 2301 (1963).

EXEMPLE 14

On opère comme à l'exemple 13, à partir de 9,4 g d'amino-2 trifluorométhoxy-6 benzothiazole et de 6,2 g d'iodure d'éthyle dans 30 cm3 d'éthanol absolu. Le mélange est chauffé 51 heures à ébullition puis refroidi à une température voisine de 20°C. Le précipité est séparé par filtration, traité par 20 cm3 de soude 1N dans 50 cm3 d'eau distillée puis extrait par 200 cm3 d'acétate d'éthyle. Après concentration à sec à 40°C sous pression réduite (20 mm de mercure ; 2,7 kPa), le résidu est repris dans 30 cm3 d'éther éthylique et traité par 3,1 cm3 d'éther chlorhydrique 4,2N. On obtient 3,5 g de chlorhydrate d'éthyl-3 imino-2 trifluorométhoxy-6 benzothiazoline fondant à 234°C.

EXEMPLE 15

On opère comme à l'exemple 13, à partir de 7 g d'amino-2 trifluorométhoxy-6 benzothiazole et de 10,2 g d'iodo-1 propane dans 20 cm3 d'éthanol absolu. Le mélange est chauffé 42 heures à ébullition. Après refroidissement à une température voisine de 20°C, le milieu réactionnel est concentré à sec à 50°C sous pression

réduite (20 mm de mercure ; 2,7 kPa) et le résidu traité par 3,2 g de carbonate de sodium dans l'eau distillée. Après extraction par 200 cm3 d'acétate d'éthyle et purification par chromatographie sur colonne de silice avec un mélange d'éther éthylique et de cyclohexane (65-35 en volumes) comme éluant, on obtient 1,3 g d'imino-2 propyl-3 trifluorométhoxy-6 benzothiazoline transformée en chlorhydrate sublimant vers 180°C.

EXEMPLE 16

On opère comme à l'exemple 13, à partir de 9,4 g d'amino-2 trifluorométhoxy-6 benzothiazole et de 9,6 g de bromure d'allyle dans 30 cm3 d'éthanol absolu. Le mélange est chauffé 48 heures à ébullition. Après refroidissement à 0°C, le précipité est filtré, lavé avec 200 cm3 d'éther éthylique et recristallisé dans 70 cm3 de propanol-2 bouillant. On obtient 4 g de bromhydrate d'allyl-3 imino-2 trifluorométhoxy-6 benzothiazoline fondant à 225°C.

EXEMPLE 17

On opère comme à l'exemple 13, à partir de 9,4 g d'amino-2 trifluorométhoxy-6 benzothiazole et de 10,8 g de bromo-4 butène-1 dans 30 cm3 de propanol-2. Le mélange est chauffé 48 heures à ébullition. Après refroidissement à une température voisine de 20°C, le précipité est filtré, puis lavé 2 fois avec 50 cm3 de propanol-2. On obtient 2,5 g de bromhydrate de (butène-3 yl)-3 imino-2 trifluorométhoxy-6 benzothiazoline fondant à 202°C.

EXEMPLE 18

On opère comme à l'exemple 13, à partir de 9,4 g d'amino-2 trifluorométhoxy-6 benzothiazole et de 12,7 g de bromométhylcyclopropane dans 30 cm3 de propanol-2. Le mélange est chauffé 42 heures à ébullition. Après refroidissement du milieu réactionnel à une température voisine de 20°C, le précipité est filtré et recristallisé dans 30 cm3 d'un mélange d'acétate d'éthyle et de méthanol (80-20 en volumes) bouillant. On obtient 1,2 g de bromhydrate de cyclopropylméthyl-3 imino-2 trifluorométhoxy-6 benzothiazoline fondant à 220°C.

EXEMPLE 19

9,4 g d'amino-2 trifluorométhoxy-6 benzothiazole, 9,7 g de N-(chloro-2 éthyl) acétamide et 13,5 g d'iodure de sodium dans 30 cm3 de méthyléthylcétone sont chauffés 40 heures à ébullition. Après refroidissement à une température voisine de 20°C, le milieu réactionnel est ajouté à 200 cm3 d'eau distillée, traité par 40 cm3 de soude 1N, puis extrait par 200 cm3 d'acétate d'éthyle. Après séchage sur sulfate de magnésium puis concentration à sec à 40°C sous pression réduite (20 mm de mercure ; 2,7 kPa), le résidu est purifié par chromatographie sur colonne de silice avec un mélange d'acétate d'éthyle et de méthanol (90-10 en volumes) comme éluant. On obtient, 2,7 g d'(acétamido-2 éthyl)-3 imino-2 trifluorométhoxy-6 benzothiazoline que l'on transforme en chlorhydrate sublimant vers 180°C.

EXEMPLE 20

On opère comme à l'exemple 13, à partir de 9,4 g d'amino-2 trifluorométhoxy-6 benzothiazole et de 14,8 g d'iodoacétamide dans 50 cm3 de méthyléthylcétone. Le mélange est chauffé 18 heures à ébullition, puis refroidi à une température voisine de 20°C. Le précipité formé est filtré, puis ajouté à 100 cm3 d'eau distillée et traité par 37 cm3 de soude 1N. L'insoluble est filtré, lavé avec 100 cm3 d'eau distillée et recristallisé dans 100 cm3 de méthanol bouillant. On obtient 6,2 g d'(imino-2 trifluorométhoxy-6 benzothiazolinyl-3 ) acétamide fondant à 228°C.

EXEMPLE 21

On opère comme à l'exemple 19, à partir de 9,4 g d'amino-2 trifluorométhoxy-6 benzothiazole, de 12 g de N,N-diéthyl chloracétamide et de 13,5 g d'iodure de sodium dans 30 cm3 de éthyléthylcétone. Le mélange est chauffé 16 heures à ébullition puis refroidi à une température voisine de 20°C. Le milieu réactionnel est ajouté à 100 cm3 d'eau distillée, traité par 50 cm3 de soude 1N, puis extrait par 150 cm3 d'acétate d'éthyle. Après séchage sur sulfate de magnésium, puis concentration à sec à 40°C sous pression réduite (20 mm de mercure ; 2,7 kPa), le résidu est purifié par chromatographie sur colonne de silice avec de l'acétate d'éthyle comme éluant. On obtient, 4,2 g de N,N-diéthyl (imino-2 trifluorométhoxy-6 benzothiazolinyl-3) acétamide que l'on

transforme en chlorhydrate fondant à 223°C.

EXEMPLE 22

On opère comme à l'exemple 19, à partir de 9,4 g d'amino-2 trifluorométhoxy-6 benzothiazole, de 13,8 g de chloro-1 phénylthio-2 éthane et de 13,5 g d'iodure de sodium dans 30 cm3 de méthyléthylcétone. Le mélange est chauffé 88 heures à ébullition puis refroidi à une température voisine de 20°C. On ajoute 250 cm3 d'éther éthylique au milieu réactionnel et on filtre le précipité formé. Le solide est mis en suspension dans 250 cm3 d'eau distillée, traité par 40 cm3 de soude 1N, puis extrait par 100 cm3 d'éther éthylique. Après séchage sur sulfate de magnésium et filtration, on ajoute 150 cm3 d'acétate d'éthyle au filtrat et on traite par 10 cm3 d'éther chlorhydrique 4N. Le précipité formé est filtré et recristallisé dans 85 cm3 de propanol-2. On obtient 5,4 g de chlorhydrate d'imino-2 (phénylthio-2 éthyl)-3 trifluorométhoxy-6 benzothiazoline fondant à 174°C.

EXEMPLE 23

On opère comme à l'exemple 13, à partir de 9,4 g d'amino-2 trifluorométhoxy-6 benzothiazole et de 10 g de bromo-2 éthanol dans 30 cm3 d'éthanol absolu. Le mélange est chauffé 95 heures à ébullition, puis refroidi à une température voisine de 20°C. Le précipité formé est filtré et lavé avec 100 cm3 d'éther éthylique. On obtient 6,4 g de bromhydrate d'(imino-2 trifluorométhoxy-6 benzothiazoline-3)-2 éthanol fondant à 219°C.

EXEMPLE 24

On opère comme à l'exemple 7, à partir de 0,5 g d'imino-2 [(pyrimidinyl-2 thio)-2 éthyl]-3 trifluorométhoxy-6 benzothiazoline et de 1 g d'acide méta-chloroperbenzoïque (à 90 % en poids) dissous dans 20 cm3 de chloroforme. Le mélange réactionnel est agité 1 heure à une température voisine de 20°C puis concentré à sec à 40°C sous pression réduite (20 mm de mercure ; 2,7 KPa). Le résidu est purifié par flash-chromatographie sur colonne de silice, sous courant d'azote, à moyenne pression (0,5-1,5 bar) avec un mélange d'acétate d'éthyle et de méthanol (95-5 en volumes) comme éluant. On isole 0,35 g d'imino-2 [(pyrimidinyl-2 sulfinyl)-2 éthyl]-3 trifluorométhoxy-6 benzothiazoline fondant à 120°C.

L'imino-2 [pyrimidinyl-2 thio)-2 éthyl]-3 trifluorométhoxy-6 benzothiazoline peut être préparé de la façon suivante : on opère comme à l'exemple 6, pour la préparation de l'imino-2 [(pyridyl-2 thio)-2 éthyl]-3 trifluorométhoxy-6 benzothiazoline, à partir de 0,8 g d'hydrure de sodium à 50 % en dispersion dans l'huile de vaseline, de 1,8 g de mercapto-2 pyrimidine, de 8 g de para-toluènesulfonate de (trifluoroacétylimino-2 trifluorométhoxy-6 benzothiazolinyl-3)-2 éthyl et de 100 cm3 de diméthylformamide. Le mélange est agité 12 heures à une température proche de 20°C. On ajoute ensuite à cette solution dans l'ordre 100 cm3 d'éthanol, 50 cm3 d'eau et 50 cm3 d'ammoniaque concentrée (10N). La solution est portée au reflux pendant une heure et refroidie à une température voisine de 20°C. On extrait cette solution par deux fois 100 cm3 de dichlorométhane, on sèche sur sulfate de magnésium anhydre, on filtre et on concentre à sec à 40°C sous pression réduite (20 mm de mercure ; 2,7 KPa). L'huile ainsi obtenue est purifiée par flash-chromatographie sur colonne de silice, sous courant d'azote à moyenne pression (0,5-1,5 bar) avec un mélange d'acétate d'éthyle et de cyclohexane (50-50 en volumes) comme éluant. On isole 2,4 g d'imino-2 [(pyrimidyl-2 thio)-2 éthyl]-3 trifluorométhoxy-6 benzothiazoline fondant à 110°C.

EXEMPLE 25

On opère comme à l'exemple 7, à partir de 2 g d'imino-2 [(pyridyl-4 thio)-2 éthyl]-3 trifluorométhoxy-6 benzothiazoline et de 0,68 g d'acide méta-chloroperbenzoïque (à 90 % en poids) dissous dans 25 cm3 d'eau et 25 cm3 de dioxanne. Le mélange réactionnel est agité 12 heures à 25°C puis concentré à sec à 40°C sous pression réduite (20 mm de mercure ; 2,7 KPa) pour éliminer le dioxanne. La solution aqueuse est amenée à un pH de 12-13 par de l'ammoniaque concentrée (10N) puis extraite par deux fois 50 cm3 de dichlorométhane. La phase organique est séchée sur sulfate de magnésium anhydre et concentrée à sec à 40°C sous pression réduite (20 mm de mercure ; 2,7 kPa). L'huile ainsi obtenue est purifiée par flash-chromatographie sur colonne de silice, sous courant d'azote à moyenne pression (0,5-1,5 bar) avec un mélange d'acétate d'éthyle et de méthanol (90-10 en volumes) comme éluant. On isole ainsi une huile qui traitée avec 0,25 g d'acide oxalique et 5 cm3 d'acétone donne directement 0,9 g d'imino-2 [(pyridyl-4 sulfinyl)-2 éthyl]-3 trifluorométhoxy-6 benzothiazoline sous forme d'oxalate fondant à 194°C.

La présente invention concerne également les médicaments constitués par au moins un composé de formule (I) ou un sel d'un tel composé à l'état pur ou sous forme d'une composition dans laquelle il est associé

à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, pilules, poudres (capsules de gélatine, cachets ou granulés. Dans ces compositions, le principe actif selon l'invention est mélangé à un ou plusieurs diluants inertes, tels que amidon, cellulose, saccharose, lactose ou silice.

Ces composition peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectables qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles dans le traitement et la prévention des phénomènes convulsifs, des troubles schizophréniques et notamment des formes déficitaires de la schizophrénie, des troubles du sommeil, des phénomènes liés à l'ischémie cérébrale et des affections neurologiques où le glutamate peut-être impliqué telles que la maladie d'Alzheimer, la maladie d'Huntington, la sclérose amyotrophique latérale et l'atrophie olivopontocérébelleuse.

Les doses dépendent de l'effet recherché, de la durée du traitement et de la voie d'administration utilisée ; elles sont généralement comprises entre 30 et 300mg par jour par voie orale pour un adulte avec des doses unitaires allant de 10 à 100mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50mg de produit actif ayant la composition suivante :

| | |
|---|---|
| - imino-2 (cyano-2 éthyl)-3 trifluorométhoxy-6 benzothiazoline .................................. | 50 mg |
| - cellulose .................................. | 18 mg |
| - lactose .................................. | 55 mg |
| - silice colloïdale .................................. | 1 mg |
| - carboxyméthylamidon sodique .................................. | 10 mg |
| - talc .................................. | 10 mg |
| - stéarate de magnésium .................................. | 1 mg |

EXEMPLE B

On prépare, selon la technique habituelle, des comprimés dosés à 50mg de produit actif ayant la composition suivante :

| | |
|---|---|
| - (imino-2 trifluorométhoxy-6 benzothiazoline-3)-2 éthanesulfonamide ........ | 50 mg |
| - lactose ........ | 104 mg |
| - cellulose ........ | 40 mg |
| - polyvidone ........ | 10 mg |
| - carboxyméthylamidon sodique ........ | 22 mg |
| - talc ........ | 10 mg |
| - stéarate de magnésium ........ | 2 mg |
| - silice colloïdale ........ | 2 mg |
| - mélange d'hydroxyméthylcellulose, glycérine, oxyde de titane (72-3,5-24,5) ........ q.s.p. | 1 comprimé pelliculé terminé à 245 mg |

EXEMPLE C

On prépare, une solution injectable contenant 10mg de produit actif avant la composition suivante :

| | |
|---|---|
| - imino-2 [(pyridyl-2 sulfinyl)-2 éthyl]-3 trifluorométhoxy-6 benzothiazoline ........ | 10 mg |
| - acide benzoïque ........ | 80 mg |
| - alcool benzylique ........ | 0,06 cm3 |
| - benzoate de sodium ........ | 80 mg |
| - éthanol à 95% ........ | 0,4 cm3 |
| - hydroxyde de sodium ........ | 24 mg |
| - propylène glycol ........ | 1,6 cm3 |
| - eau ........ q.s.p. | 4 cm3 |

**Revendications**

**Revendications pour les Etats contractants suivants : DE, GB, FR, IT, NL, SE, CH, LI, BE, AT, LU**

**1.** Composés de formule :

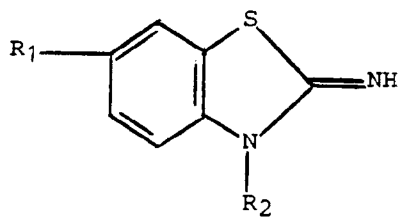

(I)

dans laquelle,

- $R_1$ représente un radical polyfluoroalcoxy et
- $R_2$ représente un radical alkyle, alcényle (3-6 C), cycloalkyl(3-6 C) alkyle, carbamoylalkyle, dialkylcarbamoylalkyle, acylaminoalkyle, phénylthioalkyle, hydroxyalkyle, cyanoalkyle, sulfamoyléthyle, N-alkylsulfamoyléthyle, pyridylthioalkyle, pyridylsulfinylalkyle, alcynyle (3-6 C), phénylsulfinylalkyle, halogénophénylthioalkyle, (trifluoro-2,2,2 éthyl) thioalkyle, pyrimidinylsulfinylalkyle, pyridylalkylsulfinylalkyle, halogénophénylsulfinylalkyle ou (trifluoro-2,2,2 éthyl) sulfinylalkyle étant entendu que les radicaux alkyle et les portions alkyle et alcoxy contiennent 1 à 4 atomes de carbone en chaîne droite ou ramifiée, ainsi que leurs sels avec un acide minéral ou organique.

2. Composés selon la revendication 1 pour lesquels $R_1$ représente un radical trifluorométhoxy, pentafluoroéthoxy, trifluoro-2,2,2 éthoxy, tétrafluoro-1,1,2,2 éthoxy ou pentafluoro-2,2,3,3,3 propoxy.

3. Procédé de préparation des composés selon la revendication 1 pour lesquels $R_2$ représente un radical cyanoalkyle, carbamoylalkyle, sulfamoyléthyle ou N-alkylsulfamoyléthyle caractérisé en ce que l'on fait agir du brome et un thiocyanate de métal alcalin sur un dérivé de formule :

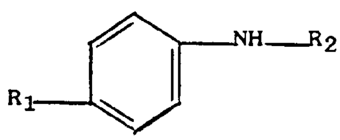

(II)

dans laquelle $R_1$ a les mêmes significations que dans la revendication 1 et $R_2$ a les mêmes significations que ci-dessus, isole le produit et le transforme éventuellement en sel d'addition avec un acide minéral ou organique.

4. Procédé de préparation des composés selon la revendication 1 pour lesquels $R_2$ représente un radical pyridylthioalkyle caractérisé en ce que l'on fait réagir un dérivé de formule :

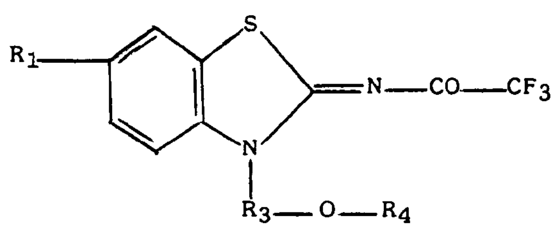

(V)

dans laquelle $R_1$ a les mêmes significations que dans la revendication 1, $R_3$ représente un radical alkylène (1-4 C) et $R_4$ représente un groupe réactif avec une mercaptopyridine, isole le produit et le transforme éventuellement en sel d'addition avec un acide minéral ou organique.

5. Procédé de préparation des composés selon la revendication 1 pour lesquels $R_2$ représente un radical alkyle, alcènyle, cycloalkylalkyle, dialkylcarbamoylalkyle, acylaminoalkyle, phénylthioalkyle, hydroxyalkyle, alcynyle, halogènophénylthioalkyle, (trifluoro-2,2,2 éthyl) thioalkyle caractérisé en ce que l'on fait réagir un dérivé aminé de formule :

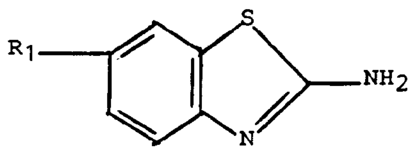

(IX)

dans laquelle $R_1$ a les mêmes significations que dans la revendication 1 sur un dérivé de formule :

$$X—R_2 \quad (X)$$

dans laquelle X représente un groupe réactif et $R_2$ a les mêmes significations que précédemment, isole le produit et le transforme éventuellement en sel d'addition avec un acide minéral ou organique.

6. Procédé de préparation des composés selon la revendication 1 pour lesquels $R_2$ représente un radical pyridylalkylsulfinylalkyle, phénylsulfinylalkyle, pyridylsulfinylalkyle, pyrimidinylsulfinylalkyle, halogénophénylsulfinylalkyle ou (trifluoro-2,2,2 éthyl) sulfinylalkyle caractérisé en ce que l'on oxyde un composé de formule (I) correspondant pour lequel $R_2$ représente un radical pyridylalkylthioalkyle, phénylthioalkyle, pyridylthioalkyle, pyrimidinylthioalkyle, halogénophénylthioalkyle ou (trifluoro-2,2,2 éthyl) thioalkyle isole le produit et le transforme éventuellement en sel d'addition avec un acide minéral ou organique.

7. Médicaments caractérisés en ce qu'ils contiennent en tant que principe actif au moins un composé selon la revendication 1 ou un sel d'un tel composé avec un acide minéral ou organique.

8. Médicaments selon la revendication 7 pour le traitement des affections ou le glutamate est impliqué.

**Revendications pour les Etats contractants suivants : ES, GR**

1. Procédé de préparation des composés formule :

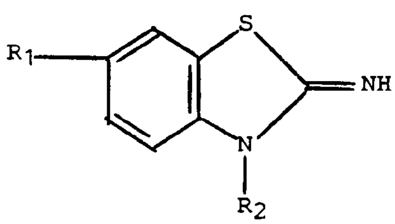

(I)

dans laquelle,

- $R_1$ représente un radical polyfluoroalcoxy et
- $R_2$ représente un radical alkyle, alcényle (3-6 C), cycloalkyl(3-6 C) alkyle, carbamoylalkyle, dialkylcarbamoylalkyle, acylaminoalkyle, phénylthioalkyle, hydroxyalkyle, cyanoalkyle, sulfamoyléthyle, N-alkylsulfamoyléthyle, pyridylthioalkyle, pyridylsulfinylalkyle, alcynyle (3-6 C), phénylsulfinylalkyle, halogénophénylthioalkyle, (trifluoro-2,2,2 éthyl) thioalkyle, pyrimidinylsulfinylalkyle, pyridylalkylsulfinylalkyle, halogénophénylsulfinylalkyle ou (trifluoro-2,2,2 éthyl) sulfinylalkyle étant entendu que les radicaux alkyle et les portions alkyle et alcoxy contiennent 1 à 4 atomes de carbone en chaîne droite ou ramifiée, ainsi que leurs sels avec un acide minéral ou organique. caractérisé en ce que :

A - pour la préparation des composés de formule (I) pour lesquels $R_2$ représente un radical cyanoalkyle, carbamoylalkyle, sulfamoyléthyle ou N-alkylsulfamoyléthyle on fait agir du brome et un thiocyanate de métal alcalin sur un dérivé de formule :

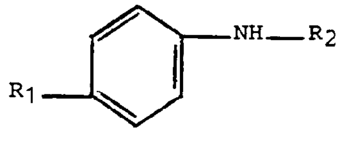

(II)

dans laquelle $R_1$ et $R_2$ ont les mêmes significations que précédemment, isole le produit et le transforme éventuellement en sel d'addition avec un acide minéral ou organique,

B - Pour la préparation des composés de formule (I) pour lesquels $R_2$ représente un radical pyridyl-

thioalkyle on fait réagir un dérivé de formule :

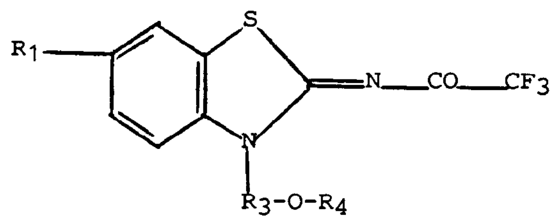

(V)

dans laquelle $R_1$ a les mêmes significations que dans la formule (I), $R_3$ représente un radical alkylène (1-4 C) et $R_4$ représente un groupe réactif avec une mercaptopyridine, isole le produit et le transforme éventuellement en sel d'addition avec un acide minéral ou organique.

C - Pour la préparation des composés de formule (I) pour lesquels $R_2$ représente un radical alkyle, alcènyle, cycloalkylalkyle, dialkylcarbamoylalkyle, acylaminoalkyle, phénylthioalkyle, hydroxyalkyle, alcynyle, halogènophénylthioalkyle, ou (trifluoro-2,2,2 éthyl) thioalkyle on fait réagir un dérivé aminé de formule :

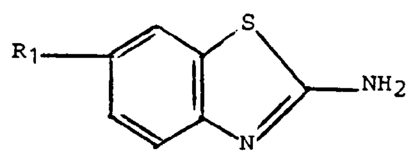

(IX)

dans laquelle $R_1$ a les mêmes significations que dans la formule (I), sur un dérivé de formule :

$$X—R_2 \quad (X)$$

dans laquelle X représente un groupe réactif et $R_2$ a les mêmes significations que précédemment, isole le produit et le transforme éventuellement en sel d'addition avec un acide minéral ou organique.

D - Pour la préparation des composés de formule (I) pour lesquels $R_2$ représente un radical pyridylalkylsulfinylalkyle, phénylsulfinylalkyle, pyridylsulfinylalkyle, pyrimidinylsulfinylalkyle, halogènophénylsulfinylalkyle ou (trifluoro-2,2,2 éthyl) sulfinylalkyle on oxyde un composé de formule (I) correspondant pour lequel $R_2$ représente un radical pyridylalkylthioalkyle, phénylthioalkyle, pyridylthioalkyle, pyrimidinylthioalkyle, halogénophénylthioalkyle ou (trifluoro-2,2,2 éthyl) trioalkyle isole le produit et le transforme éventuellement en sel d'addition avec un acide minéral ou organique.

2. Procédé selon la revendication 1 pour la préparation de composés pour lesquels $R_1$ représente un radical trifluorométhoxy, pentafluoroéthoxy, trifluoro-2,2,2 éthoxy, tétrafluoro-1,1,2,2 éthoxy ou pentafluoro-2,2,3,3 propoxy.

**Patentansprüche**

**Patentansprüche für folgende Vertragsstaaten : DE, GB, FR, IT, NL, SE, CH, LI, BE, AT, LU**

1. Verbindungen der Formel

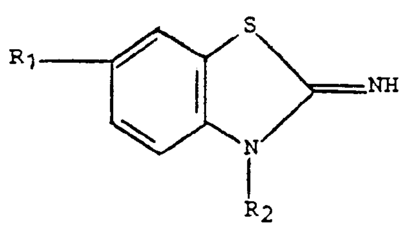

(I)

worin

$R_1$ einen Polyfluoralkoxyrest bedeutet und

$R_2$ einen Alkyl-, Alkenyl($C_{3-6}$)-, Cycloalkyl($C_{3-6}$)alkyl-, Carbamoylalkyl-, Dialkylcarbamoylalkyl-, Acylaminoalkyl-, Phenylthioalkyl-, Hydroxyalkyl-, Cyanoalkyl-, Sulfamoylethyl-, N-Alkylsulfamoylethyl-, Pyridylthioalkyl-, Pyridylsulfinylalkyl-, Alkinyl($C_{3-6}$)-, Phenylsulfinylalkyl-, Halogenophenylthioalkyl-, (2,2,2-Trifluorethyl)-thioalkyl-, Pyrimidinylsulfinylalkyl-, Pyridylalkylsulfinylalkyl-, Halogenophenylsulfinylalkyl- oder (2,2,2-Trifluorethyl)-sulfinylalkyl-Rest wiedergibt, wobei die Alkylreste und die Alkyl- und Alkoxyteile 1 bis 4 Kohlenstoffatome in gerader oder verzweigter Kette enthalten,
sowie deren Salze mit einer Mineral- oder organischen Säure.

2. Verbindungen gemäß Anspruch 1, worin $R_1$ einen Trifluormethoxy-, Pentafluorethoxy-, 2,2,2-Trifluorethoxy-, 1,1,2,2-Tetrafluorethoxy- oder 2,2,3,3,3-Pentafluorpropoxy-Rest bedeutet.

3. Verfahren zur Herstellung der Verbindungen gemäß Anspruch 1, worin $R_2$ für einen Cyanoalkyl-, Carbamoylalkyl-, Sulfamoylethyl- oder N-Alkylsulfamoylethyl-Rest steht, daduch gekennzeichnet, daß man Brom und ein Alkalimetallthiocyanat mit einem Derivat der Formel

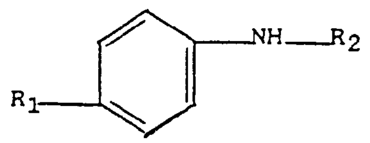

(II)

worin $R_1$ die in Anspruch 1 angegebenen Bedeutungen besitzt und $R_2$ die vorstehend angegebenen Bedeutungen hat, umsetzt, das Produkt isoliert und es gegebenenfalls in ein Additionssalz mit einer Mineral- oder organischen Säure überführt.

4. Verfahren zur Herstellung der Verbindungen gemäß Anspruch 1, worin $R_2$ für einen Pyridylthioalkylrest steht, dadurch gekennzeichnet, daß man ein Derivat der Formel

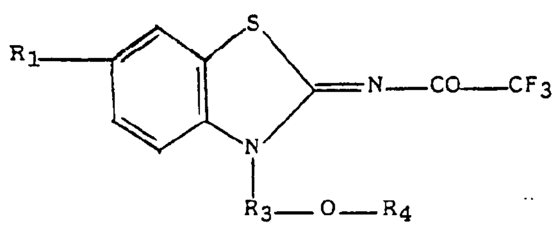

(V)

worin $R_1$ die in Anspruch 1 angegebenen Bedeutungen besitzt, $R_3$ einen Alkylen($C_{1-4}$)-Rest bedeutet und $R_4$ eine reaktive Gruppe wiedergibt, mit einem Mercaptopyridin umsetzt, das Produkt isoliert und es gegebenenfalls in ein Additionssalz mit einer Mineral- oder organischen Säure überführt.

5. Verfahren zur Herstellung der Verbindungen gemäß Anspruch 1, worin $R_2$ für einen Alkyl-, Alkenyl-, Cycloalkylalkyl-, Dialkylcarbamoylalkyl-, Acylaminoalkyl-, Phenylthioalkyl-, Hydroxyalkyl-, Alkinyl-, Halogenophenylthioalkyl-, (2,2,2-Trifluorethyl)-thioalkyl-Rest steht, dadurch gekennzeichnet, daß man ein Aminderivat der Formel

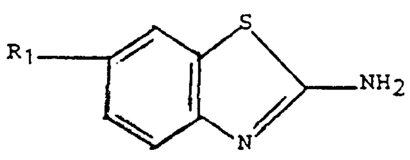

(IX)

worin $R_1$ die in Anspruch 1 angegebenen Bedeutungen besitzt, mit einem Derivat der Formel

$$X - R_2 \quad (X)$$

worin X für eine reaktive Gruppe steht und $R_2$ die vorstehend angegebenen Bedeutungen besitzt, umsetzt, das Produkt isoliert und es gegebenenfalls in ein Additionssalz mit einer Mineral- oder organischen Säure überführt.

6. Verfahren zur Herstellung der Verbindungen gemäß Anspruch 1, worin $R_2$ einen Pyridylalkylsulfinylalkyl-, Phenylsulfinylalkyl-, Pyridylsulfinylalkyl-, Pyrimidinylsulfinylalkyl-, Halogenophenylsulfinylalkyl- oder (2,2,2-Trifluorethyl)-sulfinylalkyl-Rest bedeutet, dadurch gekennzeichnet, daß man eine entsprechende Verbindung der Formel (I), worin $R_2$ für einen Pyridylalkylthioalkyl-, Phenylthioalkyl-, Pyridylthioalkyl-, Pyrimidinylthioalkyl-, Halogenophenylthioalkyl- oder (2,2,2-Trifluorethyl)-thioalkyl-Rest steht, oxidiert, das Produkt isoliert und es gegebenenfalls in ein Additionssalz mit einer Mineral- oder organischen Säure überführt.

7. Arzneimittel, daduch gekennzeichnet, daß sie als Wirkstoff zumindest eine Verbindung gemäß Anspruch 1 oder ein Salz einer derartigen Verbindung mit einer Mineral- oder organischen Säure enthalten.

8. Arzneimittel gemäß Anspruch 7 für die Behandlung von Glutamat-bedingten Erkrankungen.

**Patentansprüche für folgende Vertragsstaaten : ES, GR**

1. Verfahren zur Herstellung der Verbindungen der Formel

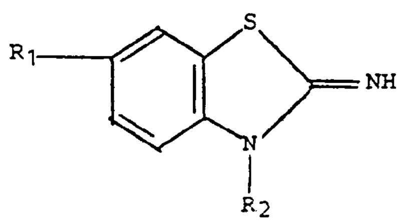

(I)

worin

$R_1$ einen Polyfluoralkoxyrest bedeutet und

$R_2$ einen Alkyl-, Alkenyl($C_{3-6}$)-, Cycloalkyl($C_{3-6}$)alkyl-, Carbamoylalkyl-, Dialkylcarbamoylalkyl-, Acylaminoalkyl-, Phenylthioalkyl-, Hydroxyalkyl-, Cyanoalkyl-, Sulfamoylethyl-, N-Alkylsulfamoylethyl-, Pyridylthioalkyl-, Pyridylsulfinylalkyl-, Alkinyl($C_{3-6}$)-, Phenylsulfinylalkyl-, Halogenphenylthioalkyl-, (2,2,2-Trifluorethyl)-thioalkyl-, Pyrimidinylsulfinylalkyl-, Pyridylalkylsulflnylalkyl-, Halogenphenylsulfinylalkyl- oder (2,2,2-Trifluorethyl)-sulfinylalkylrest wiedergibt, wobei die Alkylreste und die Alkyl- und Alkoxyteile 1 bis 4 Kohlenstoffatome in gerader oder verzweigter Kette enthalten,

sowie von deren Salzen mit einer Mineral- oder organischen Säure,

dadurch gekennzeichnet, daß man

(A) zur Herstellung der Verbindungen der Formel (I), worin $R_2$ für einen Cyanoalkyl-, Carbamoylalkyl-, Sulfamoylethyl- oder N-Alkylsulfamoylethylrest steht, Brom und ein Alkalimetallthiocyanat mit einem Derivat der Formel

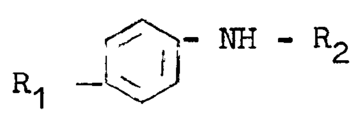

(II)

worin $R_1$ und $R_2$ die vorstehend angegebenen Bedeutungen besitzen, umsetzt, das Produkt isoliert und es gegebenenfalls in ein Additionssalz mit einer Mineral- oder organischen Säure überführt;

(B) zur Herstellung der Verbindungen der Formel (I), worin $R_2$ für einen Pyridylthioalkylrest steht, ein Derivat der Formel

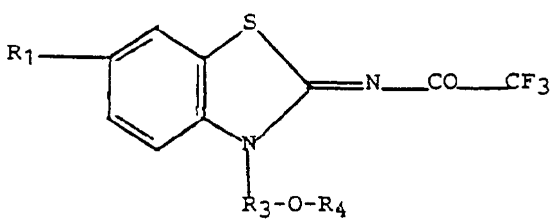

(V)

worin $R_1$ die in Formel (I) angegebenenBedeutungen besitzt, $R_3$ einen Alkylen($C_{1-4}$)-Rest wiedergibt und $R_4$ für eine reaktive Gruppe steht, mit einem Mercaptopyridin umsetzt, das Produkt isoliert und es gegebenenfalls in ein Additionssalz mit einer Mineral- oder organischen Säure überführt;

(C) zur Herstellung der Verbindungen der Formel (I), worin $R_2$ einen Alkyl-, Alkenyl-, Cycloalkylalkyl-, Dialkylcarbamoylalkyl-, Acylaminoalkyl-, Phenylthioalkyl-, Hydroxyalkyl-, Alkinyl-, Halogenophenylthioalkyl- oder (2,2,2-Trifluorethyl)-thioalkyl-Rest bedeutet, ein Aminoderivat der Formel

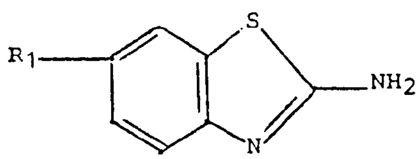

(IX)

worin $R_1$ die für Formel (I) angegebenen Bedeutungen besitzt, mit einem Derivat der Formel

$$X - R_2 \qquad (X)$$

worin X für eine reaktive Gruppe steht und $R_2$ die vorstehend angegebenen Bedeutungen besitzt, umsetzt, das Produkt isoliert und es gegebenenfalls in ein Additionssalz mit einer Mineral- oder organischen Säure überführt;

(D) zur Herstellung der Verbindungen der Formel (I), worin $R_2$ für einen Pyridylalkylsulfinylalkyl-, Phenylsulfinylalkyl-, Pyridylsulfinylalkyl-, Pyrimidinylsulfinylalkyl-, Halogenophenylsulfinylalkyl- oder (2,2,2-Trifluorethyl)-sulfinylalkyl-Rest steht, man eine entsprechende Verbindung der Formel (I), bei der $R_2$ für einen Pyridylalkylthioalkyl-, Phenylthioalkyl-, Pyridylthioalkyl-, Pyrimidinylthioalkyl-, Halogenophenylthioalkyl- oder (2,2,2-Trifluorethyl)-thioalkyl-Rest steht, oxidiert, das Produkt isoliert und es gegebenenfalls in ein Additionssalz mit einer Mineral- oder organischen Säure überführt.

2. Verfahren gemäß Anspruch 1 zur Herstellung der Verbindungen der Formel (I), worin $R_1$ für einen Trifluormethoxy-, Pentafluorethoxy-, 2,2,2-Trifluorethoxy-, 1,1, 2,2-Tetrafluorethoxy- oder 2,2,3,3-Pentafluorpropoxy-Rest steht.

## Claims

### Claims for the following Contracting States : DE, GB, FR, IT, NL, SE, CH, LI, BE, AT, LU

1. Compounds of formula:

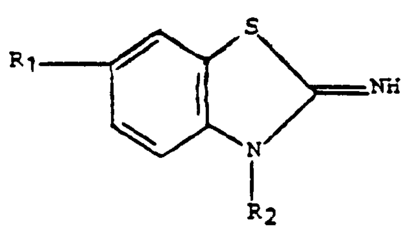

(I)

in which,

- $R_1$ represents a polyfluoroalkoxy radical and
- $R_2$ represents an alkyl, alkenyl (3-6 C), cycloalkyl(3-6 C)alkyl, carbamoylalkyl, dialkylcarbamoylalkyl, acylaminoalkyl, phenylthioalkyl, hydroxyalkyl, cyanoalkyl, sulphamoylethyl, N-alkylsulphamoylethyl, pyridylthioalkyl, pyridylsulphinylalkyl, alkynyl (3-6 C), phenylsulphinylalkyl,halophenylthioalkyl,(2,2,2-trifluoroethyl)thioalkyl, pyrimidinylsulphinylalkyl, pyridylalkylsulphinylalkyl, halophenylsulphinylalkyl or (2,2,2-trifluoroethyl)sulphinylalkyl radical, it being understood that the alkyl radicals and the alkyl and alkoxy portions contain 1 to 4 carbon atoms, in a straight- or branched-chain, as weil as their salts with an inorganic or organic acid.

2. Compounds according to claim 1 for which $R_1$ represents a trifluoromethoxy, pentafluoroethoxy, 2,2,2-trifluoroethoxy, 1,1,2,2-tetrafluoroethoxy or 2,2,3,3,3-pentafluoropropoxy radical.

3. Process of preparation of the compounds according to Claim 1 for which $R_2$ represents a cyanoalkyl, carbamoylalkyl, sulphamoylethyl or N-alkylsulphamoylethyl radical, characterised in that bromine and an alkali metal thiocyanate are reacted with a derivative of formula:

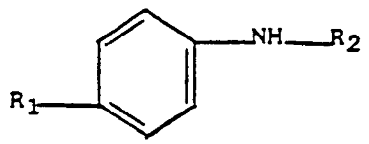

(II)

in which $R_1$ has the same meanings as in claim 1 and $R_2$ has the same meanings as above, the product is isolated and is optionally converted into an addition salt with an inorganic or organic acid.

4. Process of preparation of the compounds according to claim 1 for which $R_2$ represents a pyridylthioalkyl radical, characterised in that a reaction is carried out of a derivative of formula:

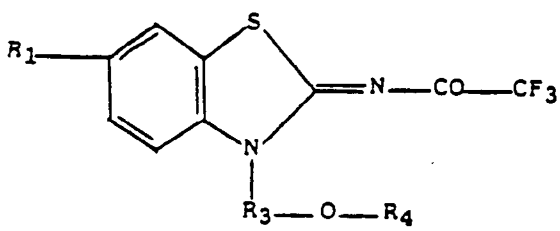

(V)

in which $R_1$ has the same meanings as in claim 1, $R_3$ represents an alkylene (1-4 C) radical and $R_4$ represents a reactive group with a mercaptopyridine, the product is isolated and is optionally converted into an addition salt with an inorganic or organic acid.

5. Process of preparation of the compounds according to claim 1 for which $R_2$ represents an alkyl, alkenyl, cycloalkylalkyl, dialkylcarbamoylalkyl, acylaminoalkyl, phenylthioalkyl, hydroxyalkyl, alkynyl, halophenylthioalkyl or (2,2,2-trifluoroethyl)thioalkyl radical, characterised in that an amino derivative of formula:

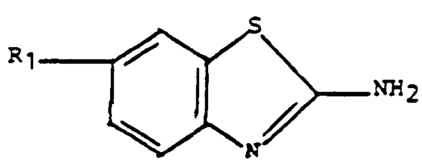

(IX)

in which $R_1$ has the same meanings as in claim 1 is reacted with a derivative of formula:

$$X—R_2 \qquad (X)$$

in which X represents a reactive group and $R_2$ has the same meanings as before, the product is isolated and is optionally converted into an addition salt with an inorganic or organic acid.

6. Process of preparation of the compounds according to claim 1 for which $R_2$ represents a pyridylalkylsulphinylalkyl, phenylsulphinylalkyl, pyridylsulphinylalkyl, pyrimidinylsulphinylalkyl, halophenylsulphinylalkyl or (2,2,2-trifluoroethyl)sulphinylalkyl radical, characterised in that a corresponding compound of formula (I) for which $R_2$ represents a pyridylalkylthioalkyl, phenylthioalkyl, pyridylthioalkyl, pyrimidinylthioalkyl, halophenylthioalkyl or (2,2,2-trifluoroethyl)thioalkyl radical is oxidised, the product is isolated and is optionally converted into an addition salt with an inorganic or organic acid.

7. Medicaments, characterised in that they contain as active principle at least one compound according to claim 1 or a salt of such a compound with an inorganic or organic acid.

8. Medicaments according to claim 7 for the treatment of ailments where glutamate is involved.

**Claims for the following Contracting States : ES, GR**

1. Process of preparation of the compounds of formula:

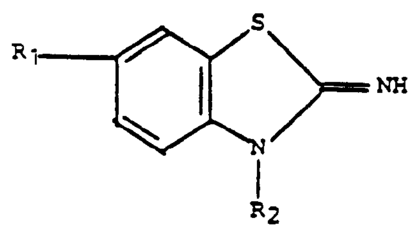

(I)

in which,

- $R_1$ represents a polyfluoroalkoxy radical and
- $R_2$ represents an alkyl, alkenyl (3-6 C), cycloalkyl(3-6 C)alkyl, carbamoylalkyl, dialkylcarbamoylalkyl, acylaminoalkyl, phenylthioalkyl, hydroxyalkyl, cyanoalkyl, sulphamoylethyl, N-alkylsulphamoylethyl, pyridylthioalkyl, pyridylsulphinylalkyl, alkynyl (3-6 C), phenylsulphinylalkyl, halophenylthioalkyl,(2,2,2-trifluoroethyl)thioalkyl, pyrimidinylsulphinylalkyl, pyridylalkylsulphinylalkyl, halophenylsulphinylalkyl or (2,2,2-trifluoroethyl)sulphinylalkyl radical, it being understood that the alkyl radicals and the alkyl and alkoxy portions contain 1 to 4 carbon atoms, in a straight- or branched-chain, as weil as their salts with an inorganic or organic acid,

characterised in that:

A - for the preparation of the compounds of formula (I) for which $R_2$ represents a cyanoalkyl, carbamoylalkyl, sulphamoylethyl or N-alkylsulphamoylethyl radical, bromine and an alkali metal thiocyanate are reacted with a derivative of formula:

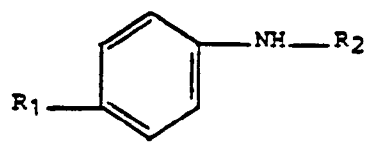

(II)

in which $R_1$ and $R_2$ have the same meanings as before, product is isolated and is optionally converted into an addition salt with an inorganic or organic acid,

B - for the preparation of the compounds of formula (I) for which $R_2$ represents a pyridylthioalkyl radical, a reaction is carried out of a derivative of formula:

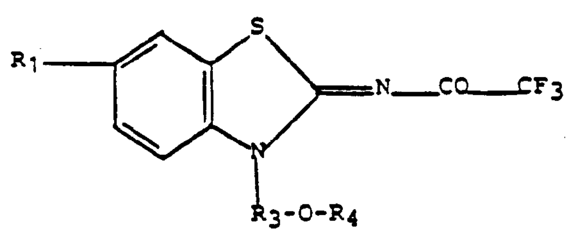

(V)

in which $R_1$ has the same meanings as in formula (I), $R_3$ represents an alkylene (1-4 C) radical and $R_4$ represents a reactive group with a mercaptopyridine, the product is isolated and is optionally converted into an addition salt with an inorganic or organic acid.

C - For the preparation of the compounds of formula (I) for which $R_2$ represents an alkyl, alkenyl, cycloalkylalkyl, dialkylcarbamoylalkyl, acylaminoalkyl, phenylthioalkyl, hydroxyalkyl, alkynyl, halophenylthioalkyl or (2,2,2-trifluoroethyl)thioalkyl radical, an amino derivative of formula:

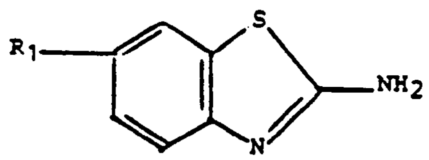

(IX)

in which $R_1$ has the same meanings as in formula (I), is reacted with a derivative of formula:

$$X—R_2 \qquad (X)$$

in which X represents a reactive group and $R_2$ has the same meanings as before, the product is isolated and is optionally converted into an addition salt with an inorganic or organic acid.

D - For the preparation of the compounds of formula (I) for which $R_2$ represents a pyridylalkylsulphinylalkyl,phenylsulphinylalkyl,pyridylsulphinylalkyl, pyrimidinylsulphinylalkyl, halophenylsulphinylalkyl or (2,2,2-trifluoroethyl)sulphinylalkyl radical, a corresponding compound of formula (I) for which $R_2$ represents a pyridylalkylthioalkyl, phenylthioalkyl, pyridylthioalkyl, pyrimidinylthioalkyl, halogenophenylthioalkyl or (2,2,2-trifluoroethyl)thioalkyl radical is oxidised, the product is isolated and is optionally converted into an addition salt with an inorganic or organic acid.

**2.** Process according to claim 1 for the preparation of compounds for which $R_1$ represents a trifluoromethoxy, pentafluoroethoxy, 2,2,2-trifluoroethoxy, 1,1,2,2-tetrafluoroethoxy or 2,2,3,3-pentafluoropropoxy radical.